# FASCICULE DE BREVET EUROPEEN

(11) **EP 0 882 697 B1**
(45) Date de publication et mention de la délivrance du brevet: **04.10.2001**
(21) Numéro de dépôt: 98109622.5
(22) Date de dépôt: 27.05.1998
(51) Int. Cl.: C07C 43/188, C11B 9/00, C07C 35/23, C07C 69/03

(54) **Composés parfumants à odeur boisée, fruitée**
Riechstoffe mit holzartigen und fruchtartigen Geruchsnoten
Perfurming agents with woody and fruity aromas

(30) Priorité: 06.06.1997 CH 137397
(43) Date de publication de la demande: 09.12.1998
(73) Titulaire: FIRMENICH SA, 1211 Genève 8 (CH)
(72) Inventeur: Schulte-Elte, Karl-Heinrich, 1213 Onex (CH); Pamingle, Hervé, 1290 Versoix (CH); Vial, Christian, 1202 Geneve (CH)
(74) Mandataire: Salvaterra-Garcia, Maria de Lurdes

(56) Documents cités:
- EP-A- 0 330 995
- US-A- 2 803 662
- J. D. WHITE: "Synthesis of dihydropallascensin via manganese(III) mediated cyclization of an olefinic beta-keto ester" TETRAHEDRON LETTERS, vol. 31, no. 1, 1990, pages 59-62, XP002075106 OXFORD GB

## Description

La présente invention a trait à des ingrédients parfumants nouveaux. Elle concerne plus particulièrement des composés de formule générale 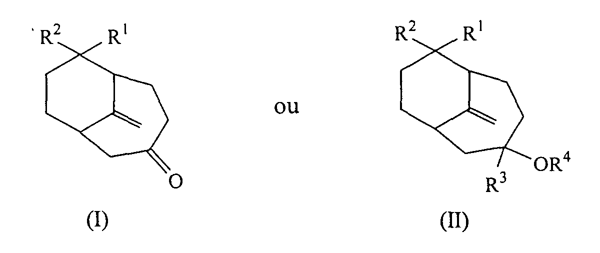 définie plus loin et leur utilisation dans la parfumerie.

Ces composés possèdent de nouvelles propriétés odorantes à savoir des odeurs boisées et, en même temps, fruitées, cette combinaison de caractéristiques olfactives n'étant pas connue parmi les composés de l'art antérieur. La demande concerne également des voies de synthèse et des produits intermédiaires nouveaux servant à préparer ces composés.

Les composés qui sont l'objet de la présente invention possèdent comme caractéristique commune le squelette bicyclique 7,7-diméthyl-10-méthylène-bicyclo [4.3.1]décane qui porte des groupes osmophores comprenant un atome d'oxygène en position exocyclique 3. Le bicycle peut, par ailleurs, comporter d'autres substituants, par exemple des groupes alkyles.

L'utilisation de substances de structure similaire, comprenant le système bicyclique 6,6-diméthyl-9-méthylène-bicyclo[3.3.1]nonane, c'est-à-dire possédant un carbone de moins dans le cycle qui comporte le groupe osmophore, est connue dans la parfumerie depuis longtemps. D'un point de vue olfactif, la substance la plus importante faisant partie de ce groupe connu est le 2-éthoxy-2,6,6-triméthyl-9-méthylène-bicyclo [3.3.1]nonane de formule 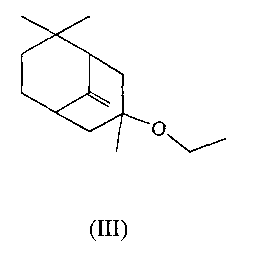 dont la synthèse a été éxécutée pour la première fois dans les années 50 par Stoll et al. (voir US-A-2,803,662). Depuis, plusieurs synthèses ont été décrites pour ce produit, toutes menant à un mélange d'isomères *exo-* et *endo*-éthoxy, avec la proportion relative entre ces deux isomères variant considérablement selon la synthèse choisie. Un mélange de ces isomères est par ailleurs en vente sous le nom commercial de Physeol (origine : Firmenich SA, Genève, Suisse). Ce composé commercial possède une odeur boisée qui est accompagnée d'une note éther ambrée.

Or, nous avons maintenant découvert que la présence d'un atome de carbone supplémentaire dans le cycle comportant le groupe osmophore résulte en des composés possédant des propriétés olfactives nettement différentes de celles du Physeol et de ses analogues, à savoir des notes boisées et ambrées doublées d'une intense note fruitée, notamment de type rhubarbe ou pamplemousse. En vue de la connaissance des propriétés des composés bicycliques de type 6,6-diméthyl-9-méthylène-bicyclo[3.3.1]nonane cités auparavant, et du Physeol en particulier, ce résultat est donc fortement inattendu et rend les composés (I) et (II) particulièrement utiles en parfumerie, de par la combinaison de caractères olfactifs citée plus haut.

L'objet de la présente invention est donc la mise à la disposition de nouveaux produits parfumants qui présentent l'odeur spécifique mentionnée ci-dessus et les synthèses de ces produits.

En conséquence, la présente demande décrit des composés de formule générale 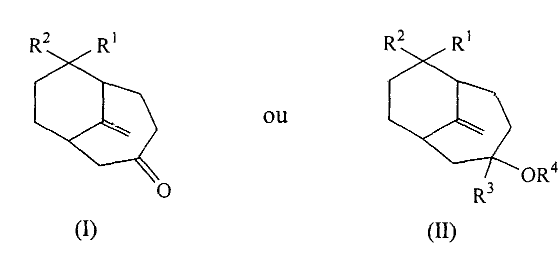 dans laquelle
R¹ et R², qui peuvent être identiques ou différents, représentent un groupe méthyle ou éthyle, de préférence un groupe méthyle,
R³ représente un atome d'hydrogène ou un groupe alkyle de C₁ à C₄, linéaire ou ramifié,
R⁴ représente un atome d'hydrogène, un groupe alkyle de C₁ à C₄, linéaire ou ramifié ou un groupe acyle de formule R⁵C(O)-, R⁵ étant un atome d'hydrogène ou un groupe alkyle de C₁ à C₄, linéaire ou ramifié,
et leur utilisation dans la parfumerie.

La caractéristique commune des composés selon la présente invention comportant le système bicyclique 7,7-diméthyl-10-méthylène-bicyclo[4.3.1]décane qui est *oxo*-substitué dans la position 3 est leur caractère olfactif. Leur odeur peut être décrite comme une note boisée qui est doublée d'une intense note fruitée. La qualité et l'intensité de cette note fruitée peut être diverse, mais la note boisée de fond est toujours nettement présente, ce qui rend ces composés particulièrement utiles en parfumerie.

Bien entendu, malgré le fait que les composés selon l'invention possèdent une odeur commune de type boisée-fruitée, il existe bien des différences, parfois très prononcées, entre les divers produits. Ces différences peuvent se révéler non seulement pour des composés de structure chimique différente, mais aussi même entre isomères stéréochimiques d'un composé donné.

Les propriétés odorantes des composés selon la présente invention se trouvent représentées au mieux dans l'acétate de 7,7-diméthyl-10-méthylène-bicyclo[4.3.1]déc-3-yle et le 3-méthoxy-7,7-diméthyl-10-méthylène-bicyclo[4.3.1.]décane. Ces deux composés sont donc préférés selon l'invention.

L'acétate de 7,7-diméthyl-10-méthylène-bicyclo[4.3.1]déc-3-yle, sous forme d'un mélange d'isomères de configuration *exo* et *endo,* développe lui une note boisée qui est doublée d'une note de type rhubarbe. L'impression globale de l'odeur de ce composé est celle d'une note boisée-racineuse de type Vetyver, le caractère racineux étant accompagné du caractère fruité mentionné. Il s'agit en fait d'un caractère olfactif très prisé en parfumerie fine.

Les deux isomères *exo* et *endo* de ce composé possèdent des odeurs distinctes. En effet, l'isomère *exo* développe une odeur où le caractère boisé, cèdre est plus marqué, alors que l'isomère *endo* possède lui une note de type boisé où les caractères rhubarbe et pamplemousse sont très prononcés.

Le 3-méthoxy-7,7-diméthyl-10-méthylène-bicyclo[4.3.1]décane se présente lui aussi sous deux formes stéréoisomères, à savoir les isomères de configuration *exo* et *endo,* qui présentent des caractéristiques olfactives différentes l'un de l'autre, et encore de celles de leur mélange. L'isomère *endo* développe une odeur de type hespéridépamplemousse, rappelant la nootkatone, qui est très puissante, alors que l'isomère *exo* présente une note boisée typique, chaude, avec un aspect rappelant l'Ambrox® (8,12-époxy-13,14,15,16-tétranorlabdane, marque enregistrée de Firmenich SA, Genève, Suisse) et l'iris. Les mélanges des deux isomères possèdent des odeurs ayant beaucoup de volume et dont les notes pamplemousse et boisée sont très substantives et tenaces. D'une façon générale, les deux isomères, ainsi que leurs mélanges, sont tous des ingrédients parfumants utiles.

Les composés répondant aux formules (I) et (II), qu'ils se présentent sous forme de l'un des isomères à l'état pur ou d'un mélange d'isomères, peuvent être utilisés aussi bien en parfumerie fine qu'en parfumerie fonctionnelle. Ils se montrent appropriés pour la préparation de compositions parfumantes diverses, de bases et concentrés parfumants, ainsi que de parfums et eaux de toilette, auxquels ils confèrent des caractères boisés-fruités. Leur utilisation dans le parfumage d'articles divers tels que les savons, gels de douche ou bain, shampooings, crèmes ou lotions après-shampooing, préparations cosmétiques ou désodorisants corporels ou d'air ambiant est aussi avantageuse.

De plus, ils conviennent également au parfumage de détergents ou adoucissants textiles et de produits d'entretien.

Les proportions dans lesquelles les composés selon la présente invention peuvent être utilisés dans les produits divers cités varient dans une gamme de valeurs très étendue. Ces valeurs sont dépendantes de la nature du produit que l'on veut parfumer et de l'effet olfactif désiré. Ils sont aussi dépendants de la nature des coingrédients dans une composition donnée, lorsque les composés selon l'invention sont utilisés en mélange avec des coingrédients parfumants, des solvants ou des adjuvants usuels dans l'art. Bien entendu, les composés selon l'invention peuvent également être ajoutés aux compositions et articles parfumés soit seuls, soit en solution dans des solvants d'usage courant.

A titre d'exemple on peut citer des concentrations de l'ordre de 1 à 10%, voire même 20% ou plus, en poids des composés selon l'invention, par rapport au poids de composition parfumante dans laquelle ils sont incorporés. Des concentrations bien inférieures à celles-ci peuvent être utilisées lorsque ces composés sont appliqués dans le parfumage des produits divers cités auparavant.

L'invention concerne également un procédé pour la préparation d'un composé de formule 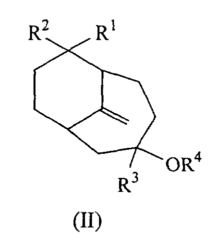 dans laquelle les symboles R¹ à R⁴ sont définis plus haut, caractérisé en ce qu'il comprend les étapes suivantes :
a) la réaction d'un composé de formule 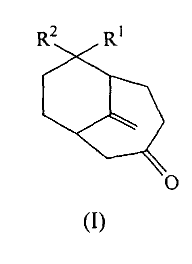 dans laquelle R¹ et R² ont le sens indiqué à la formule (II), avec un agent réducteur, pour obtenir le composé correspondant de formule (II) dans laquelle R³ et R⁴ représentent chacun un atome d'hydrogène ; ou
b) la réaction d'un composé de formule (I) avec un réactif organométallique approprié, pour former le composé de formule (II) correspondant dans lequel R³ représente un groupe alkyle de C₁ à C₄ linéaire ou ramifié et R⁴ représente un atome d'hydrogène ;
c) le cas échéant, l'éthérification du composé (II) obtenu en a) ou en b) pour former l'éther correspondant ;
d) le cas échéant, l'estérification du composé (II) obtenu en a) ou en b) pour former l'ester correspondant.

Les produits clés dans ce procédé sont la cétones cycliques (I) à partir desquelles on a accès aux produits recherchés. Ces cétones peuvent être préparées comme indiqué au schéma I. En modifiant chimiquement la fonction cétonique, on peut créer des groupes osmophores de caractères différents, ce qui donne lieu aux variations dans les caractères olfactifs des molécules formées.

On connaît de l'art antérieur un procédé analogue à celui montré au schéma I et servant à préparer la cétone de formule (I) dans laquelle R¹ et R² représentent un groupe méthyle (voir, par exemple, A. Ruveda et al., Tetrahedron 1990 (46), 4149 ; J.D. White, T.C. Somers et K.M. Yager, Tetrahedron Lett. 1990 (31), 59). Le procédé utilise comme produit de départ la α-dihydroionone, correspondant à la formule (IV) du schéma I suivant, avec R¹ = R² = méthyle. L'odeur de la cétone ainsi obtenue, ou 7,7-diméthyl-10-méthylène-bicyclo[4.3.1]décan-3-one, qui est de type boisé, avec une note rappelant le bornéol, n'a jamais été décrite dans la littérature. Cette cétone s'avère donc aussi utile en tant qu'ingrédient parfumant.

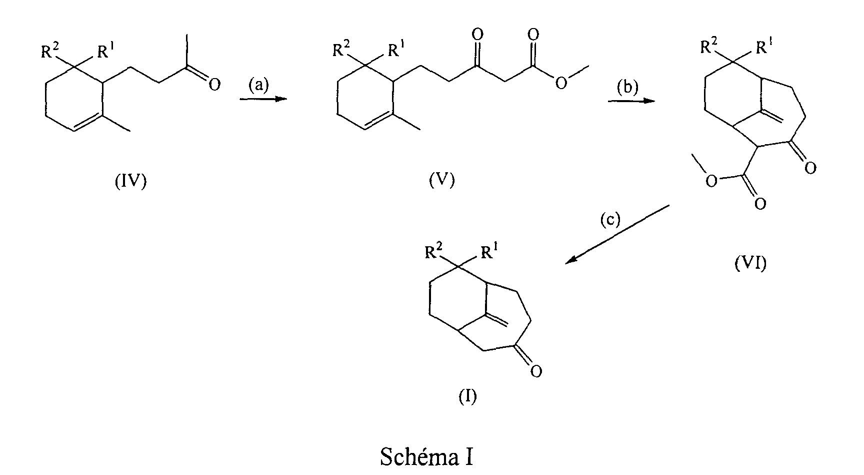

Les autres produits de départ de formule (IV) représentés au schéma I peuvent être obtenus commercialement ou facilement préparés à partir de produits commerciaux.

Le schéma II montre les possibilités de transformation des cétones de formule (I).

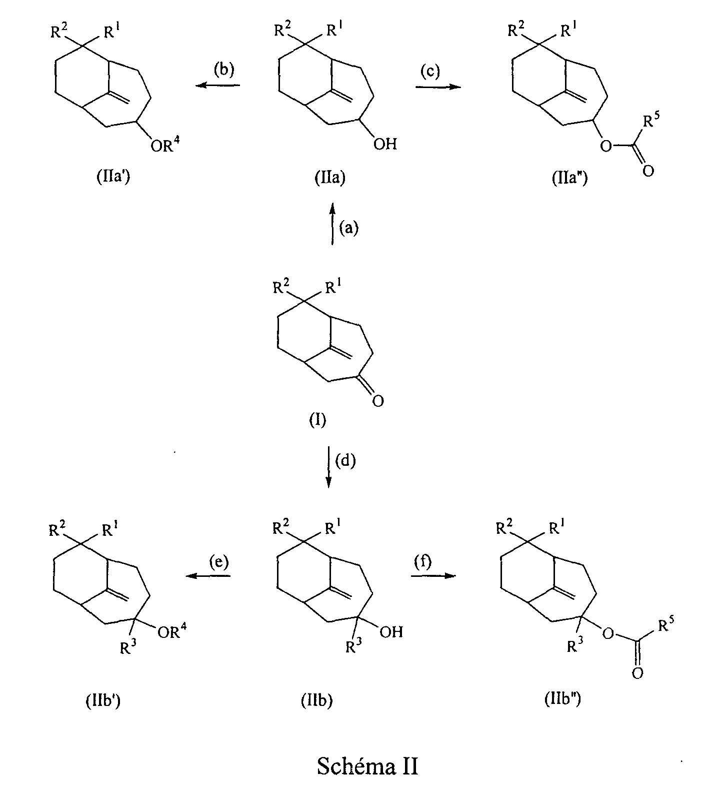

Il ressort du schéma II que les cétones (I) peuvent être directement réduites dans les alcools (IIa) correspondants à l'aide d'agents réducteurs appropriés. Dans ce contexte, on peut citer par exemple le LiAlH₄ ou le NaBH₄. Le solvant dans lequel se déroule la réduction est choisi parmi les éthers, par exemple le THF ou l'éther diéthylique ou les alcools, par exemple le méthanol ou l'éthanol. Il est évident que, en sus des agents réducteurs et solvants cités ci-dessus, il y a d'autres réactifs et solvants qui eux aussi donnent des résultats satisfaisant les exigences de la présente invention.

De la même façon, les cétones (I) sont aussi susceptibles de réagir avec un réactif organométallique (étape d) pour fournir, après l'hydrolyse, l'alcool α-substitué (IIb) correspondant. Comme agent métallant approprié, on peut citer les composés Grignard et les composés organolithiens, et d'autres agents métallants qui sont connus de l'homme de métier. La réaction est exécutée dans un solvant inerte qui, le cas échéant, est capable de stabiliser le réactif organométallique. En tant que solvant on peut utiliser un solvant courant dans ce type de réactions.

Les alcools (IIa) et (IIb) sont susceptibles d'être transformés dans les éthers (IIa') et (IIb') à l'aide d'agents d'éthérification, [voir les étapes (b) ou (e)]. Une voie convenable est la synthèse Williamson qui consiste à transformer l'alcool en un alcoolate alcalin et faire réagir celui-ci avec l'halogénure de l'alcane souhaité. Il s'est avéré approprié d'utiliser un hydrure alcalin pour la transformation du groupe alcool en alcoolate, par exemple le KH ou le NaH. Le sel alcalin étant formé, la réaction d'éthérification peut se faire par exemple avec un halogénure d'alkyle. Pour la mise en oeuvre de la réaction, on choisi un solvant inerte dans lequel la réaction procède facilement. A titre d'exemple, on peut citer le THF, ou l'éther diéthylique. Bien entendu, la réaction d'éthérification étant connue en principe, l'homme de métier est capable de trouver d'autres voies d'éthérification ainsi que des solvants qui répondent aux exigences de la présente invention.

Par ailleurs, les alcools (IIa) et (IIb) peuvent être estérifiés, [étape (c) ou (f)] à l'aide de réactifs courants, par exemple un chlorure ou anhydride de l'acide carboxylique respectif. Les solvants pouvant être utilisés dans la réaction susmentionnée sont, à l'instar des réactifs d'estérification, courants pour ce type de réaction.

L'invention sera maintenant décrite plus en détail à l'aide des exemples suivants, dans lesquels les températures sont indiquées en degrés centigrades et les abréviations ont le sens usuel dans l'art.

### Exemples

**Remarque générale :** tous les spectres RMN ont été mesurés dans le CDCl₃.

### Exemple 1

### Préparation des alcools de formule (IIb)

### Méthode générale :

Dans un ballon tricol muni d'un réfrigérant, d'un thermomètre et d'une ampoule d'introduction, on place, sous atmosphère de N₂, 2,4 g (0,1 mole) de magnésium en copeaux. On ajoute 30 ml d'éther anhydre, 1 cristal d'iode et on amorce la réaction à l'aide de quelques gouttes d'halogénure. Ensuite, on introduit goutte à goutte une solution de 0,12 mole de l'halogénure d'alkyle approprié dans 50 ml d'éther anhydre, tout en laissant réagir à reflux. A la disparition du magnésium, on introduit goutte à goutte une solution de 0,078 mole de la cétone de formule (I) appropriée, à savoir la 7,7-diméthyl-10-méthylène-bicyclo[4.3.1]décan-3-one ou l'une de ses homologues, dans 50 ml d'éther anhydre, tout en laissant réagir à reflux. On brasse le mélange réactionnel encore 2 heures à température ambiante, on le verse sur de la glace et on reprend à l'éther. Après lavage du mélange obtenu à la saumure, on sèche sur Na₂SO₄. Ensuite, on filtre, on rince, et on concentre sous vide. Le mélange est purifié par chromatographie sur SiO₂ en utilisant comme éluant un mélange cyclohexane:éther / 8:2. Le produit est obtenu par distillation sur résidus au four à boules à 10 Pa.

### a) 3,7,7-triméthyl-10-méthylène-bicyclo[4.3.1]décan-3-exo-ol halogénure d'alkyle utilisé : iodure de méthyle.

On obtient 11,2 g d'huile incolore avec un rendement de 69% de la théorie, qui est constituée exclusivement de l'isomère *exo*.

Odeur : note boisée-cèdre, avec un caractère de patchouli et légèrement de transpiration.
- **IR** (pur) :: 3480, 2940, 1450, 900 cm⁻¹
- ^{**1**}**H-RMN**(360 MHz) :: 0,90(s, 3H) ; 0,94(s, 3H) ; 1,09(m, 1H) ; 1,16(s, 3H) ; 1,38(m, 1H) ; 1,52-1,89(m, 9H) ; 2,07(t, *J*=7Hz, 1H) ; 2,51(m, 1H) ; 4,76(d, *J*=3Hz, 1H) ; 4,95(d, *J*=3Hz, 1H) δ ppm
- ^{**13**}**C-RMN**: (90 MHz) : 25,6(q) ; 28,12(q) ; 28,19(q) ; 29,4(t) ; 30,1(t) ; 32,9(q) ; 34,9(s) ; 38,2(d) ; 40,2(t) ; 43(t) ; 53,2(d) ; 73,5(s) : 111,5(t) : 154,9(s) δ ppm
- **SM** *m/z* 208: (M⁺, 4) ; 190(12) ; 175(30) ; 161(19) ; 147(30) ; 134(57) ; 119(77) ; 105(97) ; 93(97) ; 79(91) ; 69(55) ; 55(50) ; 41(100).

### b) 3-éthyl-7,7-diméthyl-10-méthylène-bicyclo[4.3.1]-3-décan-ol halogénure d'alkyle utilisé : bromure d'éthyle.

On obtient 12,8 g d'huile incolore constitué de 2 isomères *endo/exo* (1/9) avec un rendement de 74% de la théorie.

Odeur : note boisée-cèdre, avec un caractère ambré, note de transpiration plus prononcée que dans l'exemple précédent.
- **IR** (pur) :: 3450, 2950, 1660, 1450, 900 cm⁻¹
- **1H-RMN** (360 MHz) :: 0,86-0,93(m, 9H) ; 1,08(m, 1H) ; 1,41(m, 3H) ; 1,47-1,79(m, 8H) ; 1,87(m, 1H) ; 2,05(m, 1H) ; 2,7(m, 1H) ; 4,74(d, *J*=3Hz) ; 4,95(d, *J*=3Hz) δ ppm
- **SM** *m/z* 222: (M⁺, 3) ; 204(13) ; 193(32) ; 175(45) ; 148(48) ; 133(48) ; 123(98) ; 107(100) ; 93(90) ; 85(28) ; 79(90) ; 69(45) ; 57(93) ; 41(72).

### Exemple 2

### Préparation des éthers de formule (IIa')

### Méthode générale :

Dans un ballon tricol muni d'un réfrigérant, d'un thermomètre et d'une ampoule d'introduction, on place, sous atmosphère de N₂, 12 ml (0,06 mole) d'une suspension de KH dans l'huile (20%, FLUKA), et on rince trois fois avec du pentane anhydre afin d'éliminer le plus possible d'huile. Ensuite, on introduit goutte à goutte une solution de 0,049 mole d'alcool de formule (IIb) approprié, à savoir le 7,7-diméthyl-10-méthylènebicyclo[4.3.1]décan-3-ol ou l'un de ses analogues dans 48 ml de THF anhydre, on brasse le mélange réactionnel pendant 2 h, et on introduit ensuite goutte à goutte 0,81 mole de l'halogénure d'alkyle choisi. Après, on brasse encore 3 h à température ambiante. Le mélange réactionnel est versé sur de la glace, puis repris à l'éther et lavé à la saumure jusqu'à neutralité. Après avoir séché sur Na₂SO₄, on filtre et concentre sous vide.

### a) 3-méthoxy-7,7-diméthyl-10-méthylène-bicyclo[4.3.1]décane

halogénure d'alkyle utilisé : iodure de méthyle
Mélange composé de 2 isomères (29/71).
Ether méthylique ***endo*** (Pic 1 ; 29% dans le mélange).
Ether méthylique ***exo*** (Pic 2 ; 71% dans le mélange).
Après distillation sur une colonne Vigreux, on obtient 9,05 g d'une huile incolore. Rendement = 88% de la théorie.
   - **IR** (pur) :: 3080, 2920, 2800, 1640, 1450, 1100, 880 cm⁻¹
   - ^{**1**}**H-RMN** (360 MHz) :: 0,85(s, 3H, ***exo***) ; 0,87(s, 3H, ***endo***) ; 0,91(s, 3H, ***exo***) ; 0,92(s, 3H, ***endo***) ; 1,05(m, 1H) ; 1,35-2,04(m, 10H) ; 2,09(dd, *J*=3, 10Hz, 1H) ; 2,53(m, 1H, ***endo***) ; 2,61(m, 1H, ***exo***) ; 3,27(s, 3H, ***endo***) ; 3,31(s, 3H, ***exo***) ; 4,6(d, *J*=3Hz, 1H, ***endo***) ; 4,61(d, *J*=3Hz, ***exo***) ; 4,71(d, *J*=3Hz, ***endo***) ; 4,83(d, *J*=3Hz, ***exo***) δ ppm
   - ¹³**C-RMN** (90 MHz) :: 24,2(t) ; 26(t) ; 27,3(q) ; 28,2(q) ; 28,3(t) ; 31(t) ; 31,2(t) ; 34,3(t) ; 34,5(s) ; 37,3(d) ; 41,2(t) ; 51,5(d) ; 52,6(d) ; 55,9(q) ; 78,4(d) ; 82,3(d) ; 110,5(t) ; 112(t) ; 151,4(s) ; 153(s) δ ppm

Les isomères *endo* et *exo* ont été préparés à l'état pur à partir de l'isomère *endo* et *exo* de l'acétate de 7,7-diméthyl-10-méthylène-bicyclo[4.3.1]dec-3-yl (voir exemple 3) par saponification suivie d'une éthérification.

Les données analytiques étaient les suivantes :

### Ether méthylique endo :

- **IR** (pure) :: 3080, 2920, 2800, 1640, 1450, 1100, 880 cm⁻¹
- ^{**1**}**H-RMN** (360 MHz) :: 0,87(s, 3H) ; 0,92(s, 3H) ; 1,0-2,03(m, 12H) ; 2,53(m, 1H) ; 3,27(s, 3H) ; 4,6(d, *J*=3Hz, 1H) ; 4,71(d, *J=3*Hz, 1H, ) δ ppm
- ^{**13**}**C-RMN** (90 MHz):: 25,9(t) ; 28,1(t) ; 29,1(q) ; 30,2(q) ; 34,2(t) ; 34,3(t) ; 34,5(s) ; 37,3(d) ; 41,2(t) ; 52,6(d) ; 55,9(q) ; 77,4(d) ; 110,5(t) ; 153,2(s) δ ppm
- **SM** *m/z* 208: (M⁺, 0) ;19(5) ; 176(7) ; 165(3) ; 161(17) ; 147(8) ; 137(26) ; 121(69) ; 111(63) ; 107(44) ; 91(47) ; 85(100) ; 79(63) ; 67(21) ; 41(38).

### Ether méthylique exo :

- **IR** (pure) :: 3080, 2920, 2800, 1640, 1450, 1100, 880 cm⁻¹
- ^{**1**}**H-RMN** (360 MHz) :: 0,85(s, 3H) ; 0,91(s, 3H) ; 1,06(m, 1H) ; 1,36-1,92(m, 9H) ; 2,09(dd, *J*=3, 10Hz, 1H); 2,61(m, 1H); 3,31(s, 3H) ; 4,61 (d, *J*=3Hz, 1H); 4,83(d, *J*=3Hz, 1H,) δ ppm
- ^{**13**}**C-RMN** (90 MHz) :: 24,2(t) ; 27,2(q) ; 27,9(q) ; 28,3(t) ; 31,0(t) ; 31,3(t) ; 31,2(s) ; 36,7(d) ; 41,2(t) ; 51,5(d) ; 55,9(q) ; 82,3(d) ; 111,9(t) ; 151,3(s) δ ppm
- **SM** *m/z* 208: (M⁺, 5) ; 193(1) ; 176(15) ; 161(39) ; 152(20) ; 147(13) ; 137(24) ; 133(41) ; 120(100) ; 107(79) ; 91(72) ; 85(34) ; 79(100) ; 71(33) ; 67(24) ; 55(27) ; 41(51).
Odeur : décrite dans l'introduction.

### b) 3-éthoxy-7,7-diméthyl- 10-méthylène-bicyclo[4.3.1]décane

Les éthers éthyliques ont été préparés selon le procédé décrit ci-dessus en utilisant du bromure d'éthyle comme halogénure. Après distillation au four à boules à 150° et 10 Pa, on a obtenu 0,95 g d'une huile incolore.
Rendement = 89% de la théorie.
Mélange composé de 2 isomères (71/29).
Ether éthylique ***endo*** (Pic 1; 29% dans le mélange).
Ether éthylique ***exo*** (Pic 2 ; 71% dans le mélange).
Odeur : intense, boisée-cèdre avec une note de cacahuètes grillées, avec une note de fond rappelant la rhubarbe et le pamplemousse.
   - IR (pur) :: 3080, 2940, 2800, 1640, 1460, 1100, 890 cm⁻¹
   - ^{**1**}**H-RMN** (360 MHz) :: 0,84(s, 3H, **exo**) ; 0,86(s, 3H, ***endo***) ; 0,89(s, 3H, **exo**) ; 0,91(s, 3H, ***endo***) ; 1,05(large s, 1H) ; 1,15(t, *J*=7H, 3H, ***exo***) ; 1,17(t, *J*=7H, 3H, ***endo***) ; 1,31-2,01(m, 10H) ; 2,1(large s, 1H); 2,6(m, 1H, ***exo***) ; 2,68(m, 1H, ***endo***) ; 3,30-3,50(m, 2H) ; 4,59(d, *J*=3Hz, 1H, ***endo***) ; 4,62(d, *J*=3Hz, 1H, ***exo***) ; 4,82(d, *J*=3Hz, 1H, ***exo***) ; 4,89(d, *J*=3Hz, 1H, ***endo***) δ ppm
   - ^{**13**}**C-RMN** (90 MHz) :: : 15,8(q) ; 24,5(t) ; 26,1(t, ***endo***) ; 27,2(q) ; 28,1(t) ; 28,5(q) ; 31(t) ; 34,2(s) ; 35,1 (t) ; 36;7(d) ; 37,4(d, ***endo***) ; 42,4(t) ; 51,4(d) ; 52,6(d, ***endo***) ; 63,3(t) ; 63,5(t, ***endo***) ; 80,7(d) ; 110,4(t, ***endo***) ; 111,9(t) ; 151,4(s) ; 153(s, ***endo***) δ ppm
   - **SM** *m/z* 222: (M⁺, 1) ; 207(1) ; 176(9) ; 161(30) ; 147(11) ; 133(36) ; 120(44) ; 107(69) ; 99(26) ; 91(73) ; 85(39) ; 79(100) ; 67(28) ; 55(38) ; 41(47).

### Exemple 3

### Préparation des esters de formule (IIa")

### Acétate de 7,7-diméthyl-10-méthylène-bicyclo[4.3.1]déc-3-yle

Dans un ballon tricol muni d'un réfrigérant, on a placé, sous atmosphère de N₂, 0,9 g (4,6 mmoles) de 7,7-diméthyl-10-méthylène-bicyclo[4.3.1]décan-3-ol, 8 ml d'anhydride acétique et 1 goutte d'acide phosphorique. On a laissé brasser pendant la nuit à température ambiante. Ensuite, on a ajouté 50 ml d'H₂O et brassé encore une nuit à température ambiante. Le mélange obtenu a été repris à l'éther, lavé à la saumure jusqu'à neutralité et séché sur Na₂SO₄. Après filtration et concentration sous vide on a distillé au four à boules à 150°C (10 Pa).

On a obtenu 1,09 g d'une huile incolore, avec un rendement de 99% de la théorie. Le mélange obtenu était composé de 2 isomères (71/29) qui ont été séparés par chromatographie en phase gazeuse sur une colonne de type Carbowax® de 5 mètres. Odeur : décrite dans l'introduction.

### Acétate exo (Pic 1 ; 71% dans le mélange).

- **IR** (pur) :: : 3060, 2950, 1750, 1640, 1460, 1370, 1250, 1040, 900 cm⁻¹
- ^{**1**}**H-RMN** (360 MHz): 0,85(s, 3H) ; 0,9(s, 3H) ; 1,08(dd, *J*=5, 14Hz, 1H) ; 1,4(dd, *J*=4, 14Hz, 1H) ; 1,5-1,9(m, 8H), 2,0(s, 3H) ; 2,1(m, 1H) ; 2,62(m, 1 H) ; 4,64(d, *J*=3Hz, 1H) ; 4,84(d, *J*=3Hz, 1H) ; 4,89(m, 1H) δ ppm
- ^{**13**}**C-RMN** (90 MHz): : 21,6(q) ; 23,9(t) ; 27,3(q) ; 28(q) ; 28(t) ; 30,8(t) ; 32(t) ; 34,3(s) ; 36,8(d) ; 40,8(t) ; 51,4(d) ; 75,6(t) ; 112,4(t) ; 151(s) ; 170,5(s) δ ppm
- **SM** *m/z* 236: (M⁺, 0) ; 176(56) ; 161(47) ; 147(13) ; 133(48) ; 120(83) ; 105(93) ; 91(100) ; 79(82) ; 69(41) ; 55(31) ; 43(83).

### Acétate endo (Pic 2 ; 29% dans le mélange)

- **IR** (pur) :: 3060, 2950, 1750, 1640, 1460, 1370, 1250, 1040, 900 cm⁻¹
- ^{**1**}**H-RMN** (360 MHz) :: 0,87(s, 3H) ; 0,91(s, 3H) ; 1,08(d, *J*=14Hz, 1H) ; 1,39(d, *J*=14Hz, 1H) ; 1,45-2,07(m, 9H) ; 1,99(s, 3H) ; 2,58(m, 1H) ; 4,64(d,*J*=3Hz, 1H) ; 4,78(d, *J*=3Hz, 1H) ; 4,86(m, 1H) δ ppm
- ^{**13**}**C-RMN** (90 MHz) :: : 21,6(q) ; 25,7(t) ; 28,3(q) ; 28,3(q) ; 29(t) ; 30(t) ; 31,9(t) ; 34,5(s) ; 34,5(t) ; 36, 9(d) ; 52,5(d) ; 72,5(d) ; 111,6(t) ; 151,8(s) ; 170,(s) δ ppm
- **SM** *m/z* 236: (M⁺,0);194(5) ; 176(20) ; 161(40) ; 147(23) ; 136(48) ; 120(65) ; 107(90) ; 91(89) ; 79(84) ; 69(40) ; 55(36) ; 43(100).

### Example 4

### Composition parfumante

On a préparé une composition parfumante de base pour un parfum de type masculin en utilisant les ingrédients suivants :

| Ingrédients | Parties en poids |
|---|---|
| Acétate de linalyle | 350 |
| Ambrinol à 10% * ¹⁾ | 20 |
| Ambrox ® ²⁾ | 30 |
| Essence de citron | 600 |
| Coumarine | 60 |
| α-Damascone à 10% * ³⁾ | 50 |
| Dihydromyrcenol ⁴⁾ | 660 |
| Essence d'estragon | 20 |
| Farenal® à 10% * ⁵⁾ | 35 |
| 2-Méthyl-4-(2,2,3-triméthyl-3-cyclopentén-1-yl)-4-pentén-1-ol⁶⁾ | 5 |
| Galaxolide® 50 ⁷⁾ | 200 |
| Geraniol | 40 |
| Essence de géranium de Chine | 120 |
| Hédione ® ⁸⁾ | 350 |
| Essence de laurier noble | 10 |
| Linalol | 150 |
| Lyral ® ⁹⁾ | 80 |
| 1-(2,6,6-triméthyl-1-cyclohexyl)-3-hexanol à 10% * ¹⁰⁾ | 70 |
| Osyrol ® ¹¹⁾ | 130 |
| Tonalide ® ¹²⁾ | 500 |
| Vanilline | 20 |
| Total | 3500 |

| | |
|---|---|
| * dans le dipropylène glycol | |
| 1) octahydro-2,5,5-triméthyl-2-naphthalenol; origine : Firmenich SA, Genève, Suisse | |
| 2) 8,12-époxy-13,14,15,16-tétranorlabdane; origine : Firmenich SA, Genève, Suisse | |
| 3) origine : Firmenich SA, Genève, Suisse | |
| 4) 2,6-diméthyl-7-octén-2-ol ; origine : International Flavors and Fragrances Inc., USA | |
| 5) 2,6,10-triméthyl-9-undécanal ; origine : Haarmann & Reimer GmbH, Allemagne | |
| 6) origine : Firmenich SA, Genève, Suisse | |
| 7) 1,3,4,6,7,8-hexahydro-4,6,6,7,8,8-hexaméthyl-cyclopenta[g]-2-benzopyrane ; origine : International Flavors and Fragrances Inc., USA | |
| 8) dihydrojasmonate de méthyle ; origine : Firmenich SA, Genève, Suisse | |
| 9) 4-(4-hydroxy-4-méthylpentyl)-3-cyclohexène-1-carbaldéhyde et 3-(4-hydroxy-4-méthylpentyl)-3-cyclohexène-1-carbaldehyde; origine : International Flavors & Fragrances Inc., USA | |
| 10) origine : Firmenich SA, Genève, Suisse | |
| 11) 7-méthoxy-3,7-diméthyl-2-octanol ; origine : Bush, Boak Allen Ltd., Grande-Bretagne | |
| 12) 7-acétyl-1,1,3,4,4,6-hexaméthyltétraline; origine : PFW, Naarden, Pays-Bas | |

A cette composition de base hespéridée, boisée, musquée on a ajouté 1700 parties en poids de 3-méthoxy-7,7-diméthyl-10-méthylène-bicyclo[4.3.1]décane. On a ainsi obtenu une composition nouvelle dont la note boisée avait acquis une fraîcheur typique des colognes à caractère hespéridé très appréciée. Lorsqu'on a ajouté 1700 parties en poids de l'acétate de 7,7-diméthyl-10-méthylène-bicyclo[4.3.1]déc-3-yle, on a obtenu une composition nouvelle qui, en plus de sa note de fond boisée, possédait un joli côté racineux évoquant le vétyver.

### Exemple 5

### Composition parfumante

On a préparé, à partir des ingrédients suivants, une composition parfumante de base pour une eau de toilette masculine.

| Ingrédients | Parties en poids |
|---|---|
| Acétate de benzyle | 60 |
| Acétate de linalyle | 450 |
| Acétate de phényléthyle | 5 |
| Alcool cinnamique à 50% * | 30 |
| Aldéhyde amylcinnamique | 100 |
| Aldéhyde C 10 à 10% * | 40 |
| 4-(4-hydroxy-1-phényl)-2-butanone à 1% * ¹⁾ | 30 |
| Essence de bergamote | 600 |
| Essence de carvi | 70 |
| Ambrox ® à 10% ** ²⁾ | 30 |
| Citral pur | 320 |
| Phénoxyacétate d'allyle ³⁾ | 50 |
| Coumarine | 65 |
| Eugénol | 90 |
| Essence de géranium de Chine | 90 |
| Hédione ® ⁴⁾ | 200 |
| Héliopropanal ⁵⁾ | 40 |
| Indol à 10% * * * | 70 |
| Iralia ® ⁶⁾ | 80 |
| Jasmone | 5 |
| Essence de lavande | 480 |
| Linalol | 800 |
| Essence de mandarine | 100 |
| Mousse de chêne abs. du Maroc | 60 |
| Essence de patchouli | 150 |
| Essence de petitgrain | 40 |
| Essence de romarin | 30 |
| Undécalactone | 5 |
| β-Ionone ⁷⁾ | 10 |
| Total | 4100 |

| | |
|---|---|
| * dans le dipropylène glycol | |
| ** dans le 2-(2-éthoxyéthoxy)-1-éthanol; origine : Firmenich SA, Genève, Suisse | |
| * * * dans la triéthanolamine | |
| 1) origine : Firmenich SA, Genève, Suisse | |
| 2) voir exemple 4 | |
| 3) origine : Firmenich SA, Genève, Suisse | |
| 4) voir exemple 4 | |
| 5) 3-(1,3-benzodioxol-5-yl)-2-méthylpropanal; origine : Firmenich SA, Genève, Suisse | |
| 6) méthylionone ; origine : Firmenich SA, Genève, Suisse | |
| 7) origine : Firmenich SA, Genève, Suisse | |

A cette composition de base, on a ajouté 150 parties en poids de 3-méthoxy-7,7-diméthyl-10-méthylène-bicyclo[4.3.1]décane. On a alors observé un renforcement de la note boisée, et encore de façon plus marquée du caractère hespéridé dejà présent dans la composition de base.

L'addition de 150 parties en poids de l'acétate de 7,7-diméthyl-10-méthylène-bicyclo [4.3.1]déc-3-yle, a pour sa part conféré plus de volume et renforcé la note de fond boisée de la composition de base, tout en lui apportant un effet chypré résultant de l'accord du composé de l'invention avec la mousse de chêne.

### Exemple 6

### Composition parfumante

On a préparé, à partir des ingrédients suivants, une composition parfumante de base destinée à une eau de toilette masculine de type "Boisé-Fougère".

| Ingrédients | Parties en poids |
|---|---|
| Acétate de linalyle | 500 |
| Essence de bergamote | 200 |
| Citral pur | 20 |
| Essence de citron | 60 |
| Citronellol | 90 |
| Essence de civette purif. à 50% * | 20 |
| Coranol ¹⁾ | 50 |
| Coumarine | 80 |
| 2-Méthyl-4-(2,2,3-triméthyl-3-cyclopentén-1-yl)-4-pentén-1-ol ²⁾ | 30 |
| Essence de géranium de Chine | 30 |
| Hédione ® ³⁾ | 400 |
| Essence de lavandin | 180 |
| Lyral ® ⁴⁾ | 300 |
| Essence de mandarine | 60 |
| Mousse de chêne abs. du Maroc à 50% * | 40 |
| Essence de patchouli | 720 |
| Vanilline | 20 |
| Total | 2800 |

| | |
|---|---|
| * dans le dipropylène glycol | |
| 1) 4-cyclohexyl-2-méthyl-2-butanol ; origine : Firmenich SA, Genève, Suisse | |
| 2), 3), 4) voir exemple 4 | |

A cette composition de base on a ajouté 400 parties en poids de 3-méthoxy-7,7-diméthyl-10-méthylène-bicyclo[4.3.1]décane pour obtenir une composition nouvelle dont la note boisée était devenue plus fraîche et avait acquis encore plus de volume. Lorsqu'on a ajouté à cette composition de base déjà bien boisée 400 parties en poids de l'acétate de 7,7-diméthyl-10-méthylène-bicyclo[4.3.1]déc-3-yle, on lui a conféré une dimension plus racineuse, vétyver.

### Exemple 7

### Composition parfumante

On a préparé, à l'aide des ingrédients suivants, une composition parfumante pour une eau de toilette de type "oriental".

| Ingrédients | Parties en poids |
|---|---|
| Acétate de benzyle | 130 |
| Acétate de linalyle | 230 |
| Ambrinol à 10% ∗ ¹⁾ | 30 |
| Astrotone ²⁾ | 300 |
| 4-(4-hydroxy-1-phényl)-2-butanone à 1% ∗ ³⁾ | 20 |
| Ambrox ® à 10% ∗∗ ⁴⁾ | 80 |
| γ-Decalactone à 10% ⁵⁾ | 90 |
| 2-Méthyl-4-(2,2,3-triméthyl-3-cyclopentén-1-yl)-4-pentén-1-ol ⁶⁾ | 20 |
| Galaxolide ® 50 ⁷⁾ | 700 |
| Essence de géranium d'Afrique | 30 |
| Hédione ® ⁷⁾ | 930 |
| Linalol | 125 |
| Lyral ® ⁸⁾ | 310 |
| δ-Nonalactone ⁹⁾ | 10 |
| Essence de patchouli | 20 |
| Vanilline | 635 |
| Vertofix Coeur ® ¹⁰⁾ | 340 |
| Total | 4000 |

| | |
|---|---|
| ∗ dans le dipropylène glycol | |
| ∗∗ dans le 2-(2-éthoxyéthoxy)-1-éthanol ; origine : Firmenich SA, Genève, Suisse | |
| 1), 4), 6), 7), 8) voir exemple 4 | |
| 2) 1,4-dioxa-5,17-cyclohéptadécanedione ; origine : Firmenich SA, Genève, Suisse | |
| 3) voir exemple 5 | |
| 5) origine : Firmenich SA, Genève, Suisse | |
| 9) origine : Firmenich SA, Genève, Suisse | |
| 10) mélange de 9-acétyl-8-cédrène et sesquiterpène de cèdre ; origine : International Flavors and Fragrances Inc., USA | |

En ajoutant 400 parties en poids de 3-méthoxy-7,7-diméthyl-10-méthylène-bicyclo[4.3.1]-décane à cette composition de base, on lui a conféré un caractère hespéridé excellent. L'effet de ce composé de l'invention est très marquant à haute concentration, comme ici. La même quantité d'acétate de 7,7-diméthyl-10-méthylène-bicyclo[4.3.1]déc-3-yle ajoute à la composition de base un effet naturel boisé-classique par son mariage avec le patchouli.

## Revendications

1. Utilisation à titre d'ingrédient parfumant d'un composé de formule 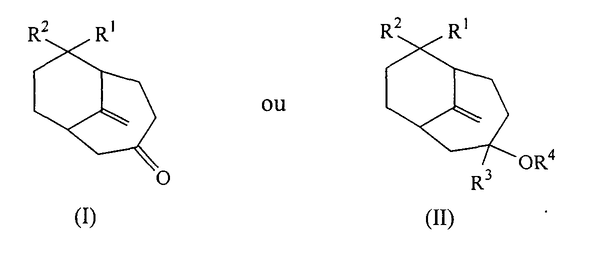 dans laquelle
R¹ et R², qui peuvent être identiques ou différents, représentent un groupe méthyle ou éthyle,
R³ représente un atome d'hydrogène ou un groupe alkyle de C₁ à C₄, linéaire ou ramifié,
R⁴ représente un atome d'hydrogène, un groupe alkyle de C₁ à C₄, linéaire ou ramifié ou un groupe acyle de formule R⁵C(O)-, R⁵ étant un atome d'hydrogène ou un groupe alkyle de C₁ à C₄, linéaire ou ramifié.

2. Utilisation selon la revendication 1, d'un composé de formule 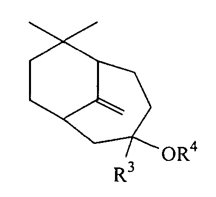 dans laquelle R³ et R⁴ sont définis comme à la revendication 1.

3. Utilisation selon la revendication 2, du 3-méthoxy-7,7-diméthyl-10-méthylène-bicyclo[4.3.1]décane ou de l'acétate de 7,7-diméthyl-10-méthylène-bicyclo[4.3.1]dèc-3-yle.

4. Utilisation selon la revendication 3, de l'exo-3-méthoxy-7,7-diméthyl-10-méthylène-bicyclo[4.3.1]décane, de l'endo-3-méthoxy-7,7-diméthyl-10-méthylène-bicyclo[4.3.1]décane, de l'acétate de l'exo-7,7-diméthyl-10-méthylène-bicyclo[4.3.1] dec-3-yle ou de l'acétate de l'endo-7,7-diméthyl-10-méthylène-bicyclo[4.3.1]dec-3-yle.

5. Composé de formule 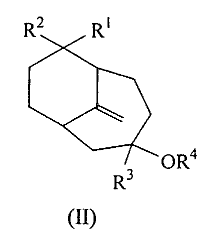 dans laquelle les symboles R¹ à R⁴ sont définis comme à la revendication 1.

6. A titre de composé selon la revendication 5, le 3-méthoxy-7, 7-diméthyl-10-méthylène-bicyclo[4.3.1]décane ou l'acétate de 7,7-diméthyl-10-méthylène-bicyclo [4.3.1]déc-3-yle.

7. A titre de composé selon la revendication 5, l'exo-3-méthoxy-7, 7-diméthyl-10-méthylène-bicyclo[4.3.1]décane, endo-3-méthoxy-7,7-diméthyl-10-méthylène-bicyclo[4.3.1]décane, l'acétate de l'exo-7,7-diméthyl-10-méthylène-bicyclo[4.3.1]déc-3-yle ou l'acétate de l'endo-7,7-diméthyl-10-méthylène-bicyclo[4.3.1]déc-3-yle.

8. Composition parfumante ou article parfumé contenant à titre d'ingrédient parfumant un composé de formule (I) ou (II) telle que définie à la revendication 1.

9. Composition parfumante ou article parfumé selon la revendication 8, contenant à titre d'ingrédient parfumant le 3-méthoxy-7,7-diméthyl-10-méthylène-bicyclo[4.3.1]décane ou l'acétate de 7,7-diméthyl-10-méthylène-bicyclo[4.3.1]déc-3-yle.

10. Composition parfumante ou article parfumé selon la revendication 9, contenant l'exo- ou l'endo-3-méthoxy-7,7-diméthyl-10-méthylène-bicyclo[4.3.1]décane ou l'acétate de l'exo- ou de l'endo-7,7- diméthyl-10-méthylène-bicyclo[4.3.1]dec-3-yle.

11. Article parfumé selon l'une des revendications 8 à 10, sous forme d'un parfum ou d'une eau de toilette, d'un savon, d'un gel de douche ou bain, d'un shampoing ou autre produit d'hygiène capillaire, d'une préparation cosmétique, d'un désodorisant corporel ou d'air ambiant, d'un détergent ou d'un adoucissant textile, ou d'un produit d'entretien.

12. Procédé pour la préparation d'un composé de formule 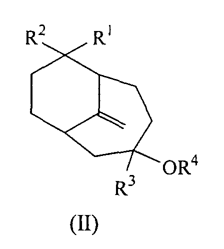 dans laquelle les symboles R¹ à R⁴ ont le sens indiqué à la revendication 1, le procédé comprenant les étapes suivantes :
a) la réaction d'un composé de formule 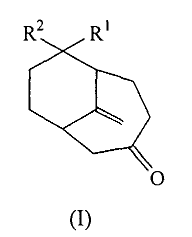 dans laquelle R¹ et R² ont le sens indiqué à la formule (II), avec un agent réducteur, pour obtenir le composé correspondant de formule (II) dans laquelle R³ et R⁴ représentent chacun un atome d'hydrogène ; ou
b) la réaction d'un composé de formule (I) avec un réactif organométallique approprié, pour former le composé de formule (II) correspondant dans lequel R³ représente un groupe alkyle de C₁ à C₄ linéaire ou ramifié et R⁴ représente un atome d'hydrogène ;
c) le cas échéant, l'éthérification du composé (II) obtenu en a) ou en b) pour former l'éther correspondant ;
d) le cas échéant, l'estérification du composé (II) obtenu en a) ou en b) pour former l'ester correspondant.

13. Utilisation d'un composé de formule 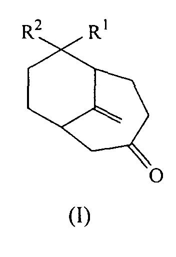 dans laquelle les symboles R ¹ et R ² ont le sens indiqué à la revendication 1, en tant que produit de départ pour la préparation des composés selon l'une des revendications 5 à 7.

## Claims

1. Use as perfuming ingredient of a compound of formula 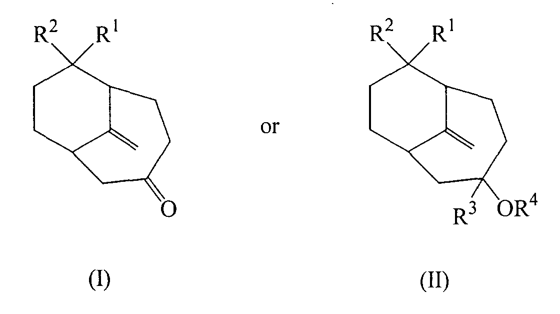 in which
R¹ and R² are identical or different and represent each a methyl or ethyl group,
R³ represents a hydrogen atom or an alkyl group from C₁ to C₄, linear or branched,
R⁴ represents a hydrogen atom, an alkyl group from C₁ to C₄, linear or branched, or an acyl group of formula R⁵C(O)-, R⁵ being a hydrogen atom or an alkyl group from C₁ to C₄, linear or branched.

2. Use according to claim 1, of a compound of formula 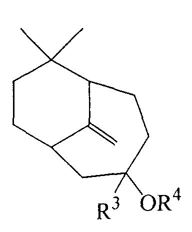 in which R³ and R⁴ are defined as in claim 1.

3. Use according to claim 2, of 3-methoxy-7,7-dimethyl-10-methylene-bicyclo[4.3.1]decane or 7,7-dimethyl-10-methylene-bicyclo[4.3.1]dec-3-yl acetate.

4. Use according to claim 3, of exo-3-methoxy-7,7-dimethyl-10-methylene-bicyclo[4.3.1]decane, endo-3-methoxy-7,7-dimethyl-10-methylene-bicyclo[4.3.1]decane, exo-7,7-dimethyl-10-methylene-bicyclo[4.3.1]dec-3-yl acetate or endo-7,7-dimethyl-10-methylene-bicyclo[4.3.1]dec-3-yl acetate.

5. A compound of formula 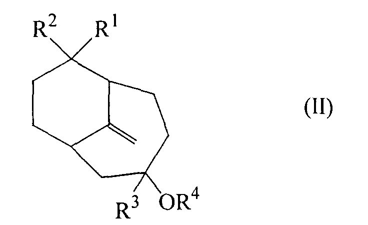 in which symbols R¹ to R⁴ are defined as in claim 1.

6. As a compound according to claim 5, 3-methoxy-7,7-dimethyl-10-methylene-bicyclo[4.3.1]decane or 7,7-dimethyl-10-methylene-bicyclo[4.3.1]dec-3-yl acetate.

7. As a compound according to claim 5, exo-3-methoxy-7,7-dimethyl-10-methylene-bicyclo[4.3.1]decane, endo-3-methoxy-7,7-dimethyl-10-methylene-bicyclo[4.3.1]decane, exo-7,7-dimethyl-10-methylene-bicyclo[4.3.1]dec-3-yl acetate or endo-7,7-dimethyl-10- methylene-bicyclo[4.3.1]dec-3-yl acetate.

8. Perfuming composition or perfumed article containing as a perfuming ingredient a compound of formula (I) or (II) as defined in claim 1.

9. Perfuming composition or perfumed article according to claim 8, containing as a perfuming ingredient 3-methoxy-7,7-dimethyl-10-methylene-bicyclo[4.3.1]decane or 7,7-dimethyl-10-methylene-bicyclo[4.3.1]dec-3-yl acetate.

10. Perfuming composition or perfumed article according to claim 9, containing exo- or endo-3-methoxy-7,7-dimethyl-10-methylene-bicyclo[4.3.1]decane or exo- or endo-7,7- dimethyl-10-methylene-bicyclo[4.3.1]dec-3-yl acetate.

11. Perfumed article according to any one of claims 8 to 10, in the form of a perfume or cologne, a soap, a bath or shower gel, a shampoo or other hair-care product, a cosmetic preparation, a body deodorant, an air-freshener, a detergent or fabric softener, or an all-purpose cleaner.

12. Process for the preparation of a compound of formula 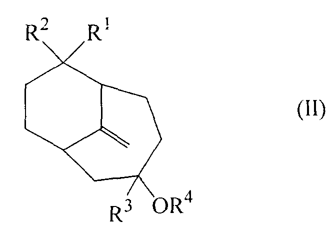 in which symbols R¹ to R⁴ have the meaning indicated in claim 1, which process comprises the following steps :
a) the reaction of a compound of formula 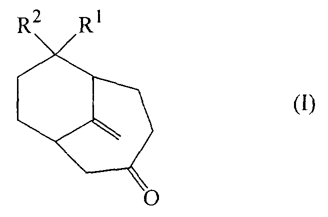 in which R¹ and R² have the meaning indicated in formula (II), with a reducing agent, to obtain the corresponding compound of formula (II) in which R³ and R⁴ each represent hydrogen; or
b) the reaction of a compound of formula (I) with an appropriate organometallic compound to form the corresponding compound of formula (II) in which R³ represents an alkyl group from C₁ to C₄, linear or branched, and R⁴ represents hydrogen ;
c) if necessary, the etherification of the compound (II) obtained in step a) or b) to form the corresponding ether;
d) if necessary, the esterification of the compound (II) obtained in step a) or b) to form the corresponding ester.

13. Use of a compound of formula 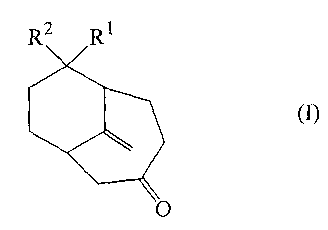 wherein the symbols R¹ and R² have the same meaning as indicated in claim 1, as the starting product for the preparation of the compounds according to claims 5 to 7.

## Patentansprüche

1. Verwendung als Riechstoff einer Verbindung der Formel 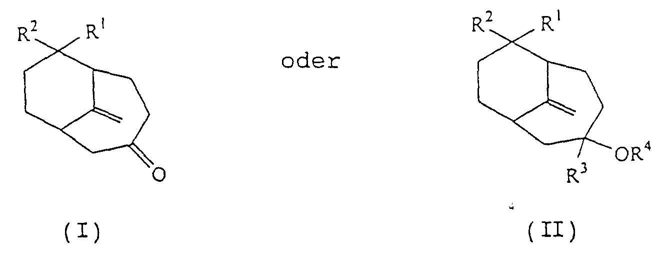 worin
R¹ und R², die identisch oder voneinander verschieden sein können, für eine Methyl- oder Ethylgruppe stehen,
R³ für ein Wasserstoffatom oder einen geradkettigen oder verzweigten C₁-C₄-Alkylrest steht,
R⁴ für ein Wasserstoffatom, einen geradkettigen oder verzweigten C₁-C₄-Alkylrest oder einen Acylrest mit der Formel R⁵C(O)- steht, wobei R⁵ ein Wasserstoffatom oder ein geradkettiger oder verzweigter C₁-C₄-Alkylrest ist.

2. Verwendung nach Anspruch 1 einer Verbindung der Formel 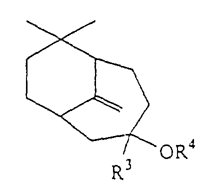 worin R³ und R⁴ wie in Anspruch 1 definiert sind.

3. Verwendung nach Anspruch 2 von 3-Methoxy-7,7-dimethyl-10-methylen-bicyclo[4.3.1]decan oder von 7,7-Dimethyl-10-methylen-bicyclo[4.3.1]dec-3-yl-acetat.

4. Verwendung nach Anspruch 3 von exo-3-Methoxy-7,7-dimethyl-10-methylen-bicyclo[4.3.1]decan, von endo-3-Methoxy-7,7-dimethyl-10-methylen-bicyclo[4.3.1]decan, von exo-7,7-Dimethyl-10-methylen-bicyclo[4.3.1]dec-3-ylacetat oder von endo-7,7-Dimethyl-10-methylen-bicyclo[4.3.1]dec-3-ylacetat.

5. Verbindung der Formel 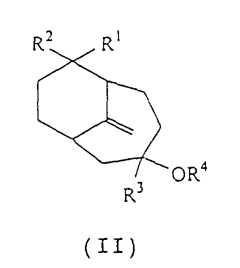 worin die Symbole R¹ bis R⁴ wie in Anspruch 1 definiert sind.

6. Verbindung nach Anspruch 5, nämlich 3-Methoxy-7,7-dimethyl-10-methylen-bicyclo[4.3.1]decan oder 7,7-Dimethyl-10-methylen-bicyclo[4.3.1]dec-3-ylacetat.

7. Verbindung nach Anspruch 5, nämlich exo-3-Methoxy-7,7-dimethyl-10-methylen-bicyclo[4.3.1]decan, endo-3-Methoxy-7,7-dimethyl-10-methylen-bicyclo[4.3.1]decan, exo-7,7-Dimethyl-10-methylen-bicyclo[4.3.1]dec-3-ylacetat oder endo-7,7-Dimethyl-10-methylen-bicyclo[4.3.1]dec-3-ylacetat.

8. Duftzusammensetzung oder parfümierter Artikel, welche bzw. welcher als Riechstoff eine Verbindung mit der Formel (I) oder (II) gemäß der Definition in Anspruch 1 enthält.

9. Duftzusammensetzung oder parfümierter Artikel nach Anspruch 8, welche bzw. welcher als Riechstoff 3-Methoxy-7,7-dimethyl-10-methylen-bicyclo[4.3.1]decan oder 7,7-Dimethyl-10-methylen-bicyclo[4.3.1]dec-3-ylacetat enthält.

10. Duftzusammensetzung oder parfümierter Artikel nach Anspruch 9, welche bzw. welcher exo- oder endo-3-Methoxy-7,7-dimethyl-10-methylen-bicyclo-[4.3.1]decan oder exo- oder endo-7,7-Dimethyl-10-methylen-bicyclo[4.3.1]dec-3-ylacetat enthält.

11. Parfümierter Artikel nach einem der Ansprüche 8 bis 10 in Form eines Parfüms oder eines Eau de Toilette, einer Seife, eines Dusch- oder Badegels, eines Shampoo oder eines anderen Haarpflegeproduktes, eines kosmetischen Präparats, eines Körper- oder Raumluft-deodorants, eines Detergens oder Textilweichspülers oder eines Allzweckreinigers.

12. Verfahren zur Herstellung einer Verbindung der Formel 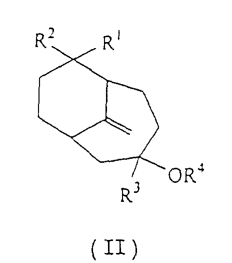 worin die Symbole R¹ bis R⁴ die in Anspruch 1 angegebene Bedeutung haben, wobei das Verfahren die folgenden Schritte umfaßt:
a) Umsetzung einer Verbindung der Formel 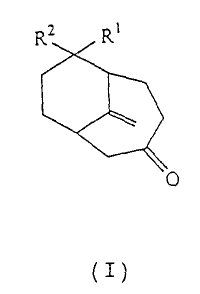 worin R¹ bis R⁴ die in Formel (II) angegebene Bedeutung haben, mit einem Reduktionsmittel zur entsprechenden Verbindung der Formel (II), worin R³ und R⁴ jeweils für ein Wasserstoffatom stehen; oder
b) Umsetzung einer Verbindung der Formel (I) mit einem geeigneten metallorganischen Reagens zur entsprechenden Verbindung der Formel (II), worin R³ für einen geradkettigen oder verzweigten C₁-C₄-Alkylrest steht und R⁴ für ein Wasserstoffatom steht;
c) gegebenenfalls Veretherung der in a) oder in b) erhaltenen Verbindung (II) zum entsprechenden Ether;
d) gegebenenfalls Veresterung der in a) oder in b) erhaltenen Verbindung (II) zum entsprechenden Ester.

13. Verwendung einer Verbindung der Formel 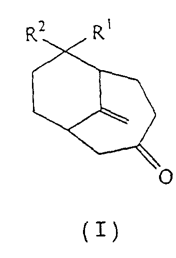 worin die Symbole R¹ und R² die in Anspruch 1 angegebene Bedeutung haben, als Ausgangsprodukt für die Herstellung der Verbindungen gemäß einem der Ansprüche 5 bis 7.
